# EUROPEAN PATENT SPECIFICATION

(11) **EP 3 313 512 B1**
(45) Date of publication and mention of the grant of the patent: **01.04.2020**
(21) Application number: 16745829.8
(22) Date of filing: 23.06.2016
(51) Int. Cl.: A61N 5/06

(54) **A DEVICE FOR LASER THERAPY**
VORRICHTUNG FÜR LASERTHERAPIE
DISPOSITIF POUR THÉRAPIES LASER

(30) Priority: 24.06.2015 IT UB20151654
(43) Date of publication of application: 02.05.2018
(73) Proprietor: Therapy Laser X SA, 6534 S. Vittore (CH)
(72) Inventor: ALGERI, Giannino, 6900 Lugano (CH)
(74) Representative: Gatti, Enrico
(86) International application number: PCT/IB2016/053740
(87) International publication number: WO 2016/207825

(56) References cited:
- WO-A2-2014/145465
- US-A1- 2014 121 653

## Description

The present invention relates to a device for laser and/or led therapy.

International scientific literature has many works that testify how electromagnetic radiations (laser and led) are responsible for the effect defined generically as biostimulation.

Reference is made to documents US2014/121653 A1 and WO2014/145465 A2.

Currently, radiation beams with high power densities are applied to surfaces to ensure statistically favourable conditions at increasing depth.

An approximate model that describes absorption as depth increases is described by the Beer-Lambert law, from which the following is deduced.

By increasing the surface power density it is possible to increase the depth of action of biostimulation.

The type of interaction with the biological tissue and the different penetration capacity, at the same power density, are influenced by the wavelength and by the absorption coefficient.

Consequently after a given depth it is not possible to obtain the desired results as it is not possible to increase the power of the source to values that could cause surface damage to the tissue.

In particular, the intensity of the radiation in relation to the irradiated surface (energy density), determines an accumulation of energy that is manifested as local thermal effect with increase of the local temperature.

Moreover, also the duration of exposure, if exceeding the critical exposure time, can cause thermal damage.

The object of the present invention is to provide a device for laser and/or led therapy that can guarantee effects on biological tissue also in depth.

Another object is to provide a device that does not generate disturbances or surface damage to the biological tissue to be treated.

A further object is to provide a device that is simple to manufacture.

Another object is to provide a device that is easy to use.

The invention is defined in the appended set of claims.

In accordance with the present invention, these and other objects are achieved by a device for laser and/or led therapy comprising: at least one laser and/or led source that emits optical radiation; an optical fibre for transfer of said optical radiation; said optical fibre is inserted into a needle, characterised in that said at least one laser and/or led source comprises three laser sources having wavelengths from 400nm to 1070nm, where at least one laser source is a therapeutic light source.

Further characteristics of the invention are described in the dependent claims..

The advantages of this solution with respect to prior art solutions are several.

The medium chosen for the optical fibre is a sterilised needle of variable size that guarantees:
1) mechanical protection against undesirable breakage of the fibres;
2) guided penetration by means of the use of CAT, ultrasound, NMR scanners or the like selected by the operator;
3) standardised dimensions that allow insertion of the fibre with calibrated stop that positions the fibre in the desired manner;
4) use of the stop prevents direct contact with the biological tissue in depth, preventing phenomena of local photocoagulation.

The new application enables a drastic decrease in the total energy transferred to the tissue. This reduction can be up to 500 times and never less than 50 times.

The laser emission is classified as having a low hazard level in class 1M.

The advantage with respect to evaluation of the stochastic (and not deterministic) risks is the reduction of the probability of occurrence.

The principles of radiation protection are mentioned. As justification, therapeutic effectiveness is obtained. As optimisation, transfer with a needle is obtained. As limitation of doses, the energy transferred is controlled via software, which prevents accidental overdoses by the operator.

The characteristics and advantages of the present invention will be apparent from the following detailed description of a practical embodiment thereof, illustrated by way of non-limiting example in the accompanying drawings, wherein:

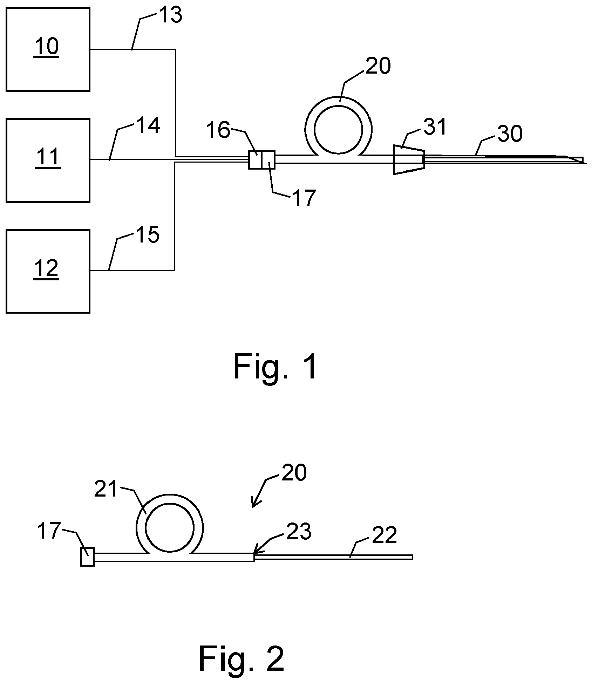

Fig. 1 schematically shows a device for laser and/or led therapy, in accordance with the present invention;
Fig. 2 schematically shows an optical fibre used in a device for laser and/or led therapy, in accordance with the present invention;

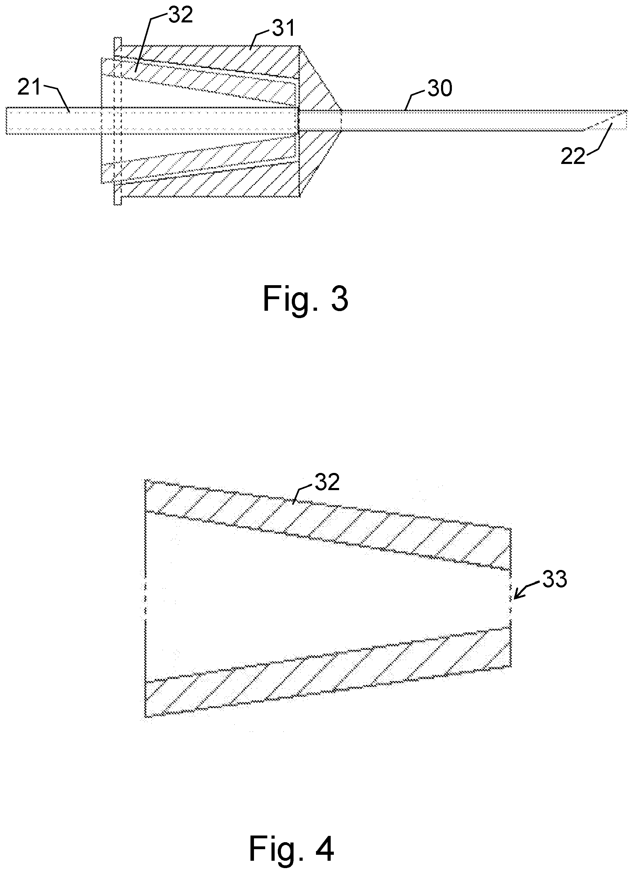

Fig. 3 schematically shows a portion of optical fibre with needle used in a device for laser and/or led therapy, in accordance with the present invention;
Fig. 4 schematically shows a conical centring instrument used in a device for laser and/or led therapy, in accordance with the present invention.

When electromagnetic radiation hits tissue it can essentially produce three effects.

Ionising, mutagenic and potentially carcinogenic effect, when the energy of the single photon is such as to be able to break the chemical bonds.

Photochemical effect, known as biostimulation effect (or rather photobiomodulation in relation to the power of some lasers to inhibit or stimulate cell functions).

Effect of increasing thermal agitation, which physically causes heating of the tissue.

The limit of separation between the possibility of obtaining or not obtaining an ionising effect is the boundary between visible light and ultraviolet. Above 400nm, even for long exposure times, there are no ionising effects, while with lower wavelengths the risk of this occurring increases, leads to the need to reduce the "critical exposure time".

In the narrow range from 400nm to 1150nm the energy associated with the single photon directly determines reactions of photochemical type: this range is called "therapeutic window".

The effects of the laser radiation, with wavelength included in the therapeutic window, result in: important mitochondrial stimulation with increase in ATP production; activation of lymphatic peristalsis; activation of microcirculation; hyperpolarisation of the membrane of the nerve fibres and consequent analgesic effect of decreased conduction of nerve stimulus; transformation of prostaglandin into prostacyclin, with blocking of the cascade of mediators and hence anti-oedema and antalgic effect.

It was also noted that over 1150nm, the water molecule starts to behave as chromophore, i.e. it completely absorbs the radiation; therefore, the interaction becomes selective, and the energy absorbed causes vibrational motions with consequent increase of the temperature of the water.

Therefore, a new therapeutic window from 450 nm to 1150 nm was selected.

Each photochemical reaction requires an exact amount of energy for two compounds to react to produce one or others.

Laser radiation is distinguished by "transporting" photons with only one energy level according to Planck's law, therefore there will be two cases to obtain the desired photochemical reaction.

If the energy required is a multiple of the energy of the photon associated with the radiation, the maximum statistical probability will be obtained, and therefore small amounts of energy are sufficient.

If the energy required is not a multiple of the energy of the photon associated with the radiation and therefore requires the involvement of photons generated by excitation and decay processes that take place in situ. This condition decreases the statistical probabilities and high levels of energy must be brought into play.

On the contrary, if the tissue is exposed simultaneously to laser radiations of different wavelengths, belonging to the therapeutic window, a simultaneous flow of photons with different energy will be obtained.

Therefore, the energy value required can be easily obtained as the sum of the combination of photons with different energies and a high statistical probability will be achieved.

The mixture of different wavelengths ensures the scattering effect even in conditions in which there is no photoconduction.

Therefore, increased stimulation and low water absorption coefficient (at the wavelengths selected) are obtained. Moreover, a new energy transfer method controlled via software was used.

The problem of reaching areas of biological tissue in depth still remains.

In accordance with the present invention a common infiltration needle is used as mechanical medium for insertion of an optical fibre into the tissue. This allows therapeutic treatments, which make use of single or multiple frequency laser therapy and/or the led therapy, to be carried out directly in depth.

This solution is particularly suitable for treating inflammation sites, oedema and all those pathological conditions that can be positively resolved through the use of laser therapy and/or led therapy quickly and without any contraindications.

The use of the needle ensures minimal invasiveness, but at the same time sterility and precision, as the user can be easily guided in the operations to penetrate the tissue with the aid of an ultrasound, CAT and NMR scanner. The use of these investigative instruments combined with the aid of the needle, of suitable length, allows the anatomical target to be reached with precision.

The solution provides for the selection of three frequencies in the range from 400nm to 1070nm, and preferably but not exclusively, a first laser source 10 at the wavelength of 635 nm, a second laser source 11 at the wavelength of 980 nm and a third laser source 12 at the wavelength of 810 nm, with total power densities that can be regulated from 0 to 0.5W/cm^2.

Alternatively, it would also be possible to use only one laser source or another number of laser sources. Moreover, led sources can be used alternatively to or in combination with the laser.

A respective optical fibre, respectively numbered 13, 14 and 15, is applied to each laser source. They are placed next to one another inside a first SMA optical connector 16, typically fixed on the device.

The means that allows application of the optical radiation in accordance with the present invention is an optical fibre 20 for delivery terminated with a second SMA optical connector 17 adapted to cooperate with the first optical connector 16.

The optical fibres 13-15 have, for example, a diameter of 100 micron. The optical fibre 20 has, for example, a diameter of 400 micron, and in any case from 100 to 600 micron.

The dimension of the optical fibre 20 must be equal to or greater than the dimension of the three optical fibres 13-15 placed next to one another to obtain efficient optical coupling.

The optical fibres 13-15 and 20 must be suitably prepared (for example the heads must be lapped perfectly perpendicular to the optical fibre) before they are fixed in the respective connectors to obtain efficient energy transfer.

The optical fibre 20 is connected to the connector 17 by means of an epoxy resin.

In order to allow the system to be re-utilised, the connector 17, the epoxy resin and the optical fibre 20 have been selected so that the characteristics of each element can guarantee that they are able to withstand being subjected to sterilisation cycles in the autoclave (at standard temperatures of 121°C), without any change in the functional parameters.

The epoxy resin is, for example, of the type 353NDPK, having an operating temperature from -55° to 250°C. The optical fibre 20 is, for example, of the type 0.39 NA TECS Hard Cladding, Step Index Multimode Optical Fiber, which has an operating temperature from - 65° to 135°C.

The optical fibre 20 has a first portion 21, of around 50 cm, starting from the connector 17, and a second portion 22, from which the protective coating 23 is removed. In the present case, with a 400 micron fibre 20, the diameter of the core is 400 micron, the diameter of the cladding is 425, and the diameter of the coating 23 is 730 micron.

Therefore, by removing the coating the diameter is reduced from 730 micron to a diameter of 425 micron.

The second portion 22 is inserted into an infiltration needle 30, which usually varies from 16 to 120 mm in length by 1.2-0.5 mm in diameter.

For example, using a needle 30 of the desired length, the internal diameter is selected so that it can pass through the second portion 22, but not the first portion 21. The variation in diameter acts as a stop to the advance of the fibre 20 in the needle 30. The length of the second portion 22 must be selected so that the optical fibre does not project from the tip of the needle 30, to avoid breaking the optical fibre, and consequent problems of recovery, and to ensure correct diffusion of the radiation. For this purpose, it is fundamental for the fibre without coating to be sufficiently long to reach the tip of the needle without passing beyond it, or at most one millimetre shorter.

Insertion of the optical fibre 20 into the needle 30, and in particular, insertion of the second portion 22, can be difficult due to the small dimensions of the objects. Therefore, a conical centring instrument has been specifically designed.

Like all needles, the needle 30 has a connector 31 for fitting it to the syringe, which in the present case is not used. A conical centring instrument 32, made of plastic, the inside of which is perforated, is inserted into the connector 31. The end bore 33 has a diameter equal to that of the inner bore of the needle 30 and, due to the thickness of the instrument 32, its outer surface comes into contact with the inner surface of the connector 31 and is thus correctly positioned.

The laser sources 10-12 are part of a treatment instrument controlled by a computer that manages all the steps of the treatment.

The new treatments introduced in "Protocol" mode have been created specifically for treating musculoskeletal disorders by means of laser application injected intra-articularly using needle-like actuators (optical fibres).

The software allows the user to choose from five different protocols, the technical parameters of which vary according to the type of treatment to be carried out.

The operator enters the password for authentication. Once it has been entered, the software launches the functions that show on the display the modes of use of the device.

The operator selects "Protocols" mode. The software launches the functions that will allow the operator to choose from five different protocols, having technical parameters created and processed specifically for intra-articular treatments.

After selecting the "Protocols" option, the system proposes a window that allows the operator to choose from several automatic management modes of the device, for which the operator does not require to set the technical parameters for emission.

Once the operator has selected the type of treatment mode desired, the software activates the connection with the actuator, and automatically sets the technical delivery parameters and application modes. It guides the operator in all steps of performing the therapy.

After inserting the needle-like optical fibre into the point to be treated, the operator enables emission by pressing the "Enable" key: pressing this key activates the device for laser emission, which takes place by pressing a foot pedal.

The treatment terminates when the energy level established by the protocol has been transferred.

The operator can carry out another treatment or log out and end the session.

Operation of the invention is apparent to those skilled in the art from the description and in particular is as follows.

The connector 17 is fitted to the connector 16.

A needle of the length required for the treatment is selected. The coating 23 is removed for the length of the needle 30.

Preferably, the instrument 32 is inserted into the connector 31 of the needle 30.

The second portion 22 is inserted into the bore of the instrument 32 (or of the needle 30) and the second portion 22 is pushed until the coating 23 of the first portion 21 abuts against the bore of the needle 30. The operator checks that the second portion 22 is not projecting from the tip of the needle.

The fibre 30 is flexible and allows the operator great freedom for manipulation.

The needle 30 is inserted into the biological tissue and the device that will guide it in its activity is activated.

At the end of the treatment the needle 30 will be disposed of while the connector 17 and the optical fibre 30 will be sterilised.

The system thus conceived is susceptible to numerous modifications and variants, all falling within the scope of the inventive concept: moreover, all the details can be replaced by technically equivalent elements.

## Claims

1. Device for laser therapy, comprising:
a needle (30); at least one laser source (10-12) configured to emit optical radiation; an optical fibre (20) for transfer of said optical radiation; said optical fibre (20) being configured to be inserted into the needle (30), **characterised in that** said at least one laser source (10-12) comprises three laser sources having wavelengths from 400nm to 1070nm, wherein at least one laser source is a therapeutic light source and said three laser sources have wavelengths of 635 nm, 980 nm and 810 nm.

2. System according to one of the preceding claims, **characterised in that** said at least one laser source (10-12) emits optical radiation having a total power density from 0 to 0.5 W/ cm².

3. System according to one of the preceding claims, **characterised in that** said optical fibre (20) comprises a protective coating (23) that is removed from said optical fibre (20) for a length equal to the length of the needle (30).

4. System according to one of the preceding claims, **characterised in that** said needle (30) comprises a connector (31) for fitting it to the syringe,
wherein said system comprises a conical centering instrument (32) to be inserted into the connector (31) of the needle.

## Patentansprüche

1. Eine Vorrichtung zur Lasertherapie, welche Folgendes umfasst: eine Nadel (30); mindestens eine
Laserquelle (10-12), die so konfiguriert ist, dass sie eine optische Strahlung abgibt; eine Lichtleitfaser (20), um die genannte optische Strahlung zu übertragen; wobei die genannte Lichtleitfaser (20) so konfiguriert ist, dass sie in die Nadel (30) eingesetzt werden kann, **dadurch gekennzeichnet, dass** die genannte mindestens eine Laserquelle (10-12) jeweils drei Laserquellen umfasst, die Wellenlängen von 400 nm bis 1070 nm aufweisen, wobei mindestens eine Laserquelle eine therapeutische Lichtquelle ist und die genannten drei Laserquellen jeweils Wellenlängen von 635 nm, 980 nm und 810 nm aufweisen.

2. Ein System gemäß einem der vorausgegangenen Ansprüche, **dadurch gekennzeichnet, dass** die genannte mindestens eine Laserquelle (10 - 12) eine optische Strahlung abgibt, welche eine Gesamtleistungsdichte von 0 bis 0.5 W/cm² aufweist.

3. Ein System gemäß einem der vorausgegangenen Ansprüche, **dadurch gekennzeichnet, dass** die genannte Lichtleitfaser (20) eine Schutzschicht (23) umfasst, die von der genannten Lichtleitfaser (20) über eine Länge entfernt wird, die jeweils der Länge der Nadel (30) entspricht.

4. Ein System gemäß einem der vorausgegangenen Ansprüche, **dadurch gekennzeichnet, dass** die genannte Nadel (30) einen Verbinder (31) umfasst, mit Hilfe dessen sie in die Spritze eingesetzt werden kann, wobei das genannte System eine entsprechende konische Zentrierungshilfe (32) umfasst, um in den Verbinder (31) der Nadel eingesetzt zu werden.

## Revendications

1. Dispositif pour la thérapie laser comprenant:
une aiguille (30); au moins une source laser (10-12) configurée pour émettre un rayonnement optique; une fibre optique (20) pour le transfert dudit rayonnement optique; ladite fibre optique (20) étant configurée pour être insérée dans l'aiguille (30), **caractérisé en ce que** ladite au moins une source laser (10-12) comprend trois sources laser ayant des longueurs d'ondes de 400 nm à 1070 nm, où au moins une source laser est une source lumineuse thérapeutique et lesdites trois sources laser ont des longueurs d'ondes de 635 nm, 980 nm et 810 nm.

2. Système selon l'une des revendications précédentes, **caractérisé en ce que** ladite au moins une source laser (10-12) émet un rayonnement optique ayant une densité de puissance totale de 0 à 0,5 W/cm².

3. Système selon l'une des revendications précédentes, **caractérisé en ce que** ladite fibre optique (20) comprend un revêtement protecteur (23) qui est retiré de ladite fibre optique (20) pour une longueur égale à la longueur de l'aiguille (30).

4. Système selon l'une des revendications précédentes, **caractérisé en ce que** ladite aiguille (30) comprend un connecteur (31) pour l'installer sur la seringue,
où ledit système comprend un instrument de centrage conique (32) pour être inséré dans le connecteur (31) de l'aiguille.
